(19) Europäisches Patentamt / European Patent Office / Office européen des brevets

(11) **EP 4 772 167 A1**

(12) **DEMANDE DE BREVET EUROPEEN**

(43) Date de publication:
08.07.2026 Bulletin 2026/28

(21) Numéro de dépôt: 25225219.2

(22) Date de dépôt: 18.12.2025

(51) Classification Internationale des Brevets (IPC):
*A61K 8/9706* (2017.01)  *A61Q 19/00* (2006.01)
*A61K 8/9711* (2017.01)

(52) Classification Coopérative des Brevets (CPC):
A61K 8/9711; A61Q 19/008

(84) Etats contractants désignés:
AL AT BE BG CH CY CZ DE DK EE ES FI FR GB GR HR HU IE IS IT LI LT LU LV MC ME MK MT NL NO PL PT RO RS SE SI SK SM TR
Etats d'extension désignés:
BA
Etats de validation désignés:
GE KH LA MA MD TN

(30) Priorité: 02.01.2025 FR 2500014

(71) Demandeur: Société d'Exploitation de Produits pour les
Industries Chimiques SEPPIC
75321 Paris cedex 07 (FR)

(72) Inventeurs:
• BIZE, Cecile
75321 Paris cedex 07 (FR)
• KERN, Catherine
75321 Paris Cedex 07 (FR)
• PECASTAINGS, Sophie
75321 Paris Cedex 07 (FR)
• LAURENCON, Lise
75321 Paris Cedex 07 (FR)

(74) Mandataire: Air Liquide
L'Air Liquide S.A.
Direction de la Propriété Intellectuelle
75, Quai d'Orsay
75321 Paris Cedex 07 (FR)

(54) **UTILISATION D'UN EXTRAIT D'UN HALOPTERIS SCOPARIA**

(57) Utilisation d'un extrait de l'algue *Halopteris scoparia* pour diminuer la quantité de sébum produite par la peau humaine et/ou par le cuir chevelu, et/ou pour prévenir et/ou pour réduire et/ou pour ralentir et/ou pour empêcher et/ou pour atténuer l'apparition des manifestations inesthétiques et/ou inconfortables et/ou désagréables de la peau et/ou du cuir chevelu chez l'être humain dues à la sécrétion de sébum, notamment dans des composition cosmétiques ou pharmaceutique, en particulier dermatologique, à usage topique.

EP 4 772 167 A1

**Description**

La présente invention concerne l'utilisation d'un extrait de l'algue *Halopteris scoparia* pour diminuer la quantité de sébum produite par la peau humaine et/ou par le cuir chevelu, et/ou pour prévenir et/ou pour réduire et/ou pour ralentir et/ou pour empêcher et/ou pour atténuer la présence de comédons fermés et/ou de comédons ouverts et/ou des papules et/ou des pustules et/ou des nodules et/ou de cicatrice et/ou de pore visiblement dilaté et/ou d'un aspect luisant ou gras ou brillant et/ou d'une sensation collante de la peau et/ou du cuir chevelu chez l'être humain dues à une sécrétion de sébum, notamment dans des compositions cosmétiques ou pharmaceutiques, en particulier dermatologique, à usage topique.

La peau humaine constituant la première image offerte au regard d'autrui, l'amélioration de son aspect est souvent un sujet de préoccupation pour l'être humain. La peau est le reflet soit d'un état de bien-être, souvent associé à une peau nette ou éclatante, soit, *a contrario,* d'un état de fatigue et/ou de négligence, souvent associé à une peau grasse ou reluisante.

La peau est un organe atypique du corps humain, extrêmement fin au regard de son étendue mais également l'organe le plus lourd d'un individu. Une des caractéristiques de la peau réside dans le fait qu'il s'agit d'un organe d'interface, d'un organe limite, entre le milieu intérieur (corps humain) et le milieu extérieur. De ce fait, et avec la flore qui la recouvre et l'habite, la peau est la première barrière de protection de l'organisme humain.

En raison de sa position d'interface avec le milieu extérieur, la peau est soumise à des sollicitations quotidiennes nombreuses, telles que par exemple le contact avec des vêtements, les changements de températures, les changements de degrés d'hygrométrie, les changements de pression, voire à des agressions, comme par exemple le contact avec certains produits chimiques présentant ou pouvant présenter un caractère très acide, ou très basique, ou irritant, avec des produits chimiques considérés comme des agents polluants.

La peau est composée de couches de différents tissus :

- L'épiderme, composé de kératinocytes, est sa partie la plus externe, puis vient
- Le derme, qui est un tissu conjonctif composé principalement de fibroblastes et de la matrice extracellulaire, et
- L'hypoderme, constitué d'adipocytes, qui est la partie la plus profonde et la plus éloignée du milieu extérieur.

La peau assure diverses fonctions dans l'intérêt de l'ensemble du système qu'elle abrite parmi lesquelles on peut retenir :

- Une fonction de barrière mécanique pour garantir l'intégrité du milieu intérieur de l'organisme,
- Une fonction émonctorielle visant à sécréter de la sueur à base d'eau, de sels et de déchets acides,
- Une fonction de régulation de la température du corps, et contient bien d'autres mécanismes de régulation, comme par exemple son mécanisme d'adaptation et de protection face aux rayonnements ultra-violets (coloration pigmentaire adaptative par la production de la mélanine), comme par exemple un système de veille immunitaire par la présence de macrophages, de cellules dendritiques.

La peau humaine constitue aussi la première image offerte au regard d'autrui. Par conséquent, l'amélioration de son aspect est un sujet de préoccupation constant pour les êtres humains. La peau est le reflet d'un état de bien-être, souvent associé à la jeunesse, et *a contrario* d'un état de fatigue et/ou de vieillissement. Il en résulte que la préservation, l'amélioration de l'état de la couche la plus extérieure de la peau, à savoir l'épiderme, constitue un centre d'intérêt majeur pour les recherches menées par les industries cosmétiques. Aussi, même une peau essentiellement saine, c'est-à-dire une peau qui se régule bien la majorité du temps, qui n'est ni trop grasse ni trop sèche, reste un organe vivant susceptible de réagir à des perturbations internes ou externes.

A la périphérie de l'épiderme, se trouve une couche cornée supérieure, nommée le stratum corneum, qui est la première couche de l'épiderme à subir les stress d'origine externe, comme les variations de conditions climatiques extérieures (température, pression, hygrométrie) ou les sollicitations mécaniques.

Le stratum corneum est plus particulièrement en contact avec le microbiote cutané.

Par « microbiote cutané », on désigne au sens de la présente demande une population de micro-organismes, spécialisés ou opportunistes, comme par exemple les bactéries, les champignons, les levures..., qui vit à la surface de la peau.

Le microbiote cutané ne peut pas être défini de façon spécifique et généralisé pour l'ensemble des individus. Depuis le lancement en 2007 du projet « Human Microbiome Project » (HMP) du National Institute of Health, les chercheurs ont pu observer de grandes variations topographiques du microbiote humain ainsi que de grandes différences selon les individus.

Au moins dix-neuf phyla ont été identifiés dont les quatre principaux sont Actinobacteria (51,8%), Firmicutes (24,4%), Proteobacteria (16,5%) et Bacteroidetes (6,3%). Les genres majoritairement identifiés sont Corynebacterium, Propionibacterium et Staphylococcus. L'abondance de chaque groupe est fortement dépendante des différentes localisations. Les organismes fongiques isolés sur la peau sont du genre Malassezia spp. Par ailleurs, des acariens du genre Demodex sont également présents et résident dans les unités pilo-sébacées, le plus souvent de la surface du visage.

Ce microbiote s'alimente à la fois de molécules excrétées par la peau (lipides, protéines...), et de composés sécrétés par les communautés de micro-organismes mettant en évidence une réelle coopération au sein de ce microbiote. De plus, cette relation avec l'hôte constitue une véritable symbiose.

Les bactéries peuvent être commensales lorsqu'elles vivent au contact du revêtement cutanéo-muqueux d'un hôte sans entraîner de dommages. Un équilibre s'installe alors entre l'individu et les flores diverses commensales de la peau et des muqueuses, mais cet équilibre est sans cesse menacé par les agressions physiques ou chimiques subies par le stratum corneum, comme par exemple la pollution, les variations de température, les rayonnements ultra-violet, l'utilisation intensive de produits tensioactifs détergents, le stress, etc.... A côté de ces bactéries commensales, se trouvent les bactéries opportunistes, indésirables et/ou pathogènes.

*Staphylococcus epidermidis (S. epidermidis)* constitue plus de 90% de la flore résidente en aérobie présente dans le stratum corneum. La flore résidente est aussi composée de bactéries anaérobies appartenant à la division des Actinobactéries comme *Propionibacterium acnes* (*P. acnes*), fréquemment retrouvés au niveau des zones sébacées, comme par exemple le dos, le visage et le cuir chevelu.

Alors que la flore normale de la peau constitue une défense pour l'hôte, une augmentation ou une réduction de la composition bactérienne (dysbiose) conduit à l'inflammation cutanée et peut être à l'origine du développement de certaines pathologies cutanées comme l'acné, la dermatite atopique ou même le psoriasis.

La peau grasse est liée à un phénomène biologique appelé hyperséborrhée ou hyper-sécrétion sébacée, définie comme étant une production anormalement élevée de sébum par la glande sébacée de la peau (hyperproduction de sébum). Ce sébum, s'écoulant à la surface de la peau, lui confère ainsi cette caractéristique disgracieuse « luisante ». Cette hyperséborrhée est généralement liée à une présence en excès de l'hormone testostérone qui, une fois dans la glande sébacée, est transformée par la 5-alpha réductase en dihydrotestostérone (DHT), qui se lie aux récepteurs présents dans les cellules sébacées et active la synthèse de sébum.

L'hyperséborrhée s'accompagne d'une hyperkératinisation folliculaire, caractérisée par une augmentation du nombre de kératinocytes au niveau de l'infundibulum folliculaire, qui provoque une obstruction du canal folliculaire et rend difficile l'écoulement du sébum.

Ceci entraîne une accumulation du sébum qui est profitable au développement de la bactérie anaérobie *Propionibacterium acnes.* Cette dernière va entraîner l'apparition d'un phénomène inflammatoire. L'ensemble de ces phénomènes conduit à l'apparition de signes cliniques caractéristiques, tels que des comédons, caractérisant les peaux acnéiques.

La présence de la bactérie P *acnes* au niveau de la structure pilo-sébacée est connue pour être impliquée dans le développement de la pathologie. L'acné vulgaire est une maladie de la peau qui est liée à une dysbiose, i.e. un déséquilibre du microbiote cutané. Une consommation excessive de tabac et d'alcool semble également appartenir aux facteurs susceptibles d'accentuer les effets de l'acné vulgaris. Parmi les autres facteurs environnementaux le stress, la pollution urbaine ainsi que le soleil (rayonnement UV induisant la peroxydation du sébum et en particulier du squalène), sont également reconnus comme étant impliqués dans l'aggravation des symptômes de l'acné vulgaris.

La colonisation du follicule pilo-sébacé par P *acnes* est un facteur important pour la réaction inflammatoire dans l'acné vulgaire. De fait, l'acné n'est pas sensu stricto une maladie infectieuse car ce germe exerce d'abord une action inflammatoire, liée à ses très nombreuses sécrétions enzymatiques et chimiques et aux réactions immunologiques qu'il provoque. Ainsi, *P. acnes* stimule la production par les sébocytes, les kératinocytes et les leucocytes (lymphocytes et monocytes) de nombreuses cytokines inflammatoires (IL-1$\alpha$, IL1$\beta$, IL-6, IL-8, IL-10, IL-12, IL-17, IL-18, TNF-$\alpha$, GM-CSF et IFN-$\gamma$) ainsi que des peptides antimicrobiens (défensines et cathélicidines), des métalloprotéinases matricielles, des espèces réactives de l'oxygène et d'autres produits impliqués dans la réaction inflammatoire. De plus, *P. acnes* sécrète une lipase qui hydrolyse les triglycérides du sébum en acides gras libres qui sont irritants et chimiotactiques pour les neutrophiles.

Parmi les extraits de végétaux que l'on peut utiliser pour leurs actions sur le microbiote humain, on peut citer un extrait lyophilisé de feuilles de bardane (ou *Arctium lappa)* pour lequel une activité antibactérienne a été mise en évidence et plus particulièrement une activité contre des micro-organismes oraux, se montrant plus efficaces contre des bactéries associées à des pathogènes endodontiques tels que *Bacillus subtilis, Candida albicans, Lactobacillus acidophilus* et *Pseudomonas aeruginosa).*

*Halopteris scoparia* est une algue brune, de l'ordre *Sphacelariale migula,* de la famille *Stypocaulaceae,* du genre *Halopteris,* de l'espèce *scoparia.* Elle se présente sous la forme de touffes de 10 à 20 centimètres de haut, très ramifiées.

Dans le cadre de leurs recherches concernant de nouveaux principes actifs pour la prévention et/ou le traitement de l'apparition des manifestations inesthétiques et/ou inconfortables et/ou désagréables de la peau et/ou du cuir chevelu, les inventeurs se sont attachés à développer une nouvelle solution technique fondée sur un extrait de l'algue *Halopteris scoparia* pour présenter des effets de diminution ou de régulation de sébum, en particulier d'une quantité en excès de sébum, sur la peau et/ou le cuir chevelu chez l'être humain, en particulier des effets dermopurifiants ou séborégulateurs.

Selon un premier aspect, l'invention a pour objet l'utilisation cosmétique par voie topique d'un extrait d'*Halopteris scoparia* comme principe actif cosmétique pour prévenir et/ou pour réduire et/ou pour ralentir et/ou pour empêcher et/ou pour atténuer la présence de comédons fermés et/ou de comédons ouverts et/ou des papules et/ou des pustules et/ou des nodules et/ou de cicatrice et/ou de pore visiblement dilaté et/ou d'un aspect luisant ou gras ou brillant et/ou d'une sensation collante dues à une sécrétion de sébum d'une peau essentiellement saine.

Selon un aspect particulier de la présente invention, l'extrait d'*Halopteris scoparia* comprenant pour 100% de sa masse :

- De 80% massique à 95% massique d'eau, de préférence de 85% massique à 90% massique, encore plus préférentiellement de 87% massique à 89% d'eau ;
- De 4,5% massique à 15% massique, de préférence de 6% massique à 12% massique, encore plus préférentiellement de 8% massique à 10% massique d'un solvant organique choisi parmi les éléments du groupe constitué par le 1,2-propanediol, le 1,3-propanediol, le 1,4-butanediol, le 1,3-butanediol, le 1,2-butanediol, le 1,2-pentanediol, le 2-méthyl-2,4-pentanediol, le 1,6-hexanediol, le 1,8-octanediol, et d'un mélange de ces composés ;
- De 0,1% massique à 5% massique, de préférence de 0,5% massique à 4% massique, plus préférentiellement de 1% massique à 3% massique, encore plus préférentiellement de 1,2% massique à 1,5% massique de matière sèche.

Selon un aspect particulier de la présente invention, le solvant organique présent dans l'extrait d'*Halopteris scoparia* peut être un mélange du 1,3-propanediol et du 1,2-pentanediol.

Selon un aspect particulier de la présente invention, le solvant organique peut comprendre pour 100% de sa masse, un mélange de deux diols différents l'un de l'autre en proportion massique égale, c'est-à-dire 50 % massique d'un premier diol et 50% massique d'un second diol différent du premier diol.

Selon un aspect particulier de la présent invention, l'extrait d'*Halopteris scoparia* peut être obtenu par un procédé comprenant les étapes successives suivantes :

- une étape a) de séchage et de broyage de l'algue *Halopteris scoparia* de façon à obtenir une biomasse ;
- une étape b) de mise en contact de la biomasse obtenue à l'étape a) avec ledit solvant organique de façon à obtenir un mélange, ledit mélange comprenant pour 100% de sa masse, de 5% massique à 15% massique de la biomasse et de 85% massique à 95% massique dudit solvant organique ;
- une étape c) d'agitation du mélange obtenu à l'étape b) pendant une durée comprise entre 1 heure et 6 heures, à une température comprise entre 15°C et 35°C ;
- une étape d) de séparation de la biomasse du mélange obtenu à l'issue de l'étape c) pour isoler une phase liquide qui correspond à l'extrait d'*Halopteris scoparia.*

Selon un aspect particulier de la présente invention, les manifestations inesthétiques et/ou inconfortables et/ou désagréables de la peau et/ou du cuir chevelu chez l'être humain dues à la sécrétion de sébum sont choisi parmi la présence des comédons fermés (points blancs ou microkystes) et/ou des comédons ouverts (points noirs) et/ou des papules et/ou des pustules et/ou des nodules et/ou des cicatrices et/ou des pores visiblement dilatés et/ou l'aspect luisant ou gras ou brillant et/ou la sensation collante.

Par « manifestations inesthétiques et/ou inconfortables et/ou désagréables de la peau et/ou du cuir chevelu due à la sécrétion de sébum », on entend au sens de l'invention, toutes modifications de l'aspect extérieur de la peau et/ou du cuir chevelu, comme par exemple, des signes visibles des modifications de l'aspect extérieur et/ou des sensations inconfortables et/ou désagréables de la peau et/ou du cuir chevelu due à la sécrétion ou la production de sébum, en particulier dues à la présence d'une quantité en excès de sébum sur la peau et/ou le cuir chevelu, comme par exemples, la présence des comédons fermés (points blancs ou microkystes) et/ou des comédons ouverts (points noirs) et/ou des papules et/ou des pustules et/ou des nodules et/ou des cicatrices et/ou des pores visiblement dilatés et/ou l'aspect luisant ou gras ou brillant et/ou la sensation collante.

Au sens de la présente invention, par « excès de sébum sur la peau et/ou le cuir chevelu », on entend une teneur en sébum sur la peau supérieure à 95 $\mu$grammes.cm$^{-2}$ ou plus particulièrement supérieure à 120 $\mu$grammes.cm$^{-2}$, mesuré avec un sébumètre.

Par « agent dermopurifiant », on entend au sens de la présente invention une substance chimique ou une composition chimique ou un extrait issue de la biomasse d'une plante dont la propriété ou la fonction technique consiste à prévenir et/ou réduire et/ou ralentir et/ou empêcher et/ou atténuer l'apparition des manifestations inesthétiques et/ou inconfortables et/ou désagréables de la peau et/ou du cuir chevelu chez l'être humain dues à la sécrétion ou à la production de sébum.

Selon un aspect particulier de la présente invention, l'extrait *d'Halopteris scoparia* est présent dans une composition cosmétique, destinée à une application par voie topique, comprenant en outre au moins un ingrédient cosmétiquement acceptable.

Par « séboragulateur », on entend au sens de la présente invention une substance chimique ou une composition chimique ou un extrait de biomasse végétale qui est capable de participer à la régulation de l'activité des glandes sébacées par la prévention et/ou la diminution directe de la sécrétion de sébum, comme par exemple le gluconate de zinc et l'acide azélaïque.

Selon un aspect particulier de la présente invention, l'extrait *d'Halopteris scoparia* est présent en tant que principe actif dans ladite composition cosmétique à usage topique comprenant en quantité cosmétiquement efficace, en particulier en une teneur comprise de 0,01% massique à 5,00% massique, ou de 0,1% massique à 1% massique, par rapport au poids total de la composition.

La présente invention a également pour objet une composition cosmétique à usage topique comprenant en tant que principe actif une quantité cosmétiquement efficace d'un extrait d'*Halopteris scoparia* et au moins un ingrédient cosmétiquement acceptable. En particulier, ladite composition cosmétique comprend pour 100% de sa masse, de 0,01% massique à 5,00% massique, ou de 0,1% massique à 1% massique, en particulier 1% massique, de l'extrait d'*Halopteris scoparia* tel que défini ci-dessus.

L'expression « à usage topique » utilisée dans la définition de la composition cosmétique objet de la présent invention, signifie que ladite composition est sous une forme adaptée pour une administration par voie topique, formulée pour permettre son application sur les peaux, en particulier la peau et le cuir chevelu, qu'il s'agisse d'une application directe ou d'une application indirecte lorsque ladite composition est intégrée par exemple dans le cas d'un produit de soin corporel sous forme de lingette en textile ou en papier ou de produits sanitaires destinés à être en contact avec les peaux, en particulier la peau et le cuir chevelu.

Par « quantité cosmétiquement efficace » de l'extrait d'algues *Halopteris scoparia,* on désigne une quantité qui permette à la peau et au cuir chevelu chez l'être humain, d'être protégés contre l'apparition des manifestations inesthétiques et/ou inconfortables et/ou désagréables de la peau et/ou du cuir chevelu chez l'être humain dues à la sécrétion de sébum, en particulier choisis parmi la présence des comédons fermés (points blancs ou microkystes) et/ou des comédons ouverts (points noirs) et/ou des papules et/ou des pustules et/ou des nodules et/ou des cicatrices et/ou des pores visiblement dilatés et/ou l'aspect luisant ou gras ou brillant et/ou la sensation collante.

Selon un aspect plus particulier de l'invention, la composition cosmétique objet de la présente invention comprend pour 100% de sa masse :

- de 0,01% à 5,00% massique de l'extrait d'*Halopteris scoparia* tel que défini ci-dessus, et
- de 95,00% à 99,99% massique d'au moins un ingrédient cosmétiquement acceptable.

Selon un aspect plus particulier de l'invention, la composition cosmétique objet de la présente invention comprend pour 100% de sa masse :

- de 0,03% à 4,00% massique de l'extrait d'*Halopteris scoparia* tel que défini ci-dessus, et
- de 96,00% à 99,97% massique d'au moins un ingrédient cosmétiquement acceptable.

Selon un aspect plus particulier de l'invention, la composition cosmétique objet de la présente invention comprend pour 100% de sa masse :

- de 0,05% à 3,00% massique de l'extrait d'*Halopteris scoparia* tel que défini ci-dessus, et
- de 97,00% à 99,95% massique d'au moins un ingrédient cosmétiquement acceptable.

Selon un aspect plus particulier de l'invention, la composition cosmétique objet de la présente invention comprend pour 100% de sa masse :

- de 0,10% à 2,50% massique de l'extrait d' *Halopteris scoparia* tel que défini ci-dessus, et
- de 97,50% à 99,90% massique d'au moins un ingrédient cosmétiquement acceptable.

Selon un aspect plus particulier de l'invention, la composition cosmétique objet de la présente invention comprend pour 100% de sa masse :

- de 0,30% à 2,00% massique de l'extrait d'*Halopteris scoparia* tel que défini ci-dessus, et
- de 98,00% à 99,70% massique d'au moins un ingrédient cosmétiquement acceptable.

Selon un aspect plus particulier de l'invention, la composition cosmétique objet de la présente invention comprend pour 100% de sa masse :

- de 0,50% à 1,50% massique de l'extrait d'*Halopteris scoparia* tel que défini ci-dessus, et
- de 98,50% à 99,50% massique d'au moins un ingrédient cosmétiquement acceptable.

Selon un aspect plus particulier de l'invention, la composition cosmétique objet de la présente invention comprend pour 100% de sa masse :

- de 0,90% à 0,99% massique de l'extrait d'*Halopteris scoparia* tel que défini ci-dessus, et
- de 99,01% à 99,10% massique d'au moins un ingrédient cosmétiquement acceptable.

Selon un aspect plus particulier de l'invention, la composition cosmétique objet de la présente invention comprend pour 100% de sa masse :

- 1% massique de l'extrait d'*Halopteris scoparia* tel que défini ci-dessus, et
- 99% massique d'au moins un ingrédient cosmétiquement acceptable.

La composition cosmétique comprenant l'extrait d'*Halopteris scoparia* lselon l'invention est en particulier destinée à prévenir et/ou à réduire et/ou à ralentir et/ou à empêcher et/ou à atténuer l'apparition des manifestations inesthétiques et/ou inconfortables et/ou désagréables de la peau et/ou du cuir chevelu chez l'être humain dues à la sécrétion de sébum, en particulier choisis parmi la présence des comédons fermés (points blancs ou microkystes) et/ou des comédons ouverts (points noirs) et/ou des papules et/ou des pustules et/ou des nodules et/ou des cicatrices et/ou des pores visiblement dilatés et/ou l'aspect luisant ou gras ou brillant et/ou la sensation collante.

La composition cosmétique peut être présente sous la forme d'une solution aqueuse ou hydro-alcoolique ou hydro-glycolique, sous forme d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile, ou sous forme d'une poudre.

La composition cosmétique objet de la présente invention peut être conditionnée dans un flacon, dans un dispositif de type "flacon" pompe, un dispositif aérosol sous forme pressurisée, dans un dispositif muni d'une paroi ajourée comme une grille ou dans un dispositif muni d'un applicateur à billes (ou aussi dit « roll-on »).

Dans le cadre de l'invention, « ingrédient cosmétiquement acceptable » est un additif chimique et/ou biologique habituellement mis en œuvre dans le domaine des formulations cosmétiques à usage topique.

De façon général, l'extrait d'*Halopteris scoparia* objet de la présente invention peut être associé à des additifs chimiques et/ou biologique habituellement mis en œuvre dans le domaine des formulations à usage topique, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les eaux thermales ou minérales, les agents plastifiants, les agents émulsionnants et co-émulsionnants, les agents opacifiants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou le cuir chevelu ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes, les pro-biotiques.

Comme exemples de tensioactifs moussants et/ou détergents que l'on peut associer à l'extrait d'*Halopteris scoparia* dans la composition cosmétique, objet de la présente invention telle que définie précédemment, on peut citer les tensioactifs moussants et/ou détergents anioniques, cationiques, amphotères ou non ioniques.

Parmi les tensioactifs anioniques moussants et/ou détergents que l'on peut associer à l'extrait d'*Halopteris scoparia* dans la composition cosmétique, objet de la présente invention telle que définie précédemment, on peut citer les sels de métaux alcalins, de métaux alcalino-terreux, d'ammonium, d'amines, ou d'aminoalcools d'alkylether sulfates, d'alkyl sulfates, d'alkylamidoéther sulfates, d'alkylaryl polyéthersulfates, de monoglycérides sulfates, d'alphaoléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkyl sulfosuccinates, d'alkyléther sulfosuccinates, d'alkylamide sulfosuccinates, d'alkyl sulfoacétates, d'alkyl sarcosinates, d'acyl iséthionates, de N-acyl taurates, d'acyl lactylates, de dérivés N-acylés d'acides aminés, de dérivés N-acylés de peptides, de dérivés N-acylés de protéines, de dérivés N-acylés d'acides gras.

Parmi les tensioactifs amphotères moussants et/ou détergents, l'on peut associer à l'extrait d'*Halopteris scoparia* dans la composition cosmétique, objet de la présente invention telle que définie précédemment, on peut citer les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents que l'on peut associer à l'extrait d'*Halopteris*

*scoparia* dans la composition cosmétique, objet de la présente invention telle que définie précédemment, on peut citer particulièrement les dérivés d'ammoniums quaternaires.

Parmi les tensioactifs non ioniques moussants et/ou détergents que l'on peut associer à l'extrait d'*Halopteris scoparia* dans la composition cosmétique, objet de la présente invention telle que définie précédemment, on peut citer plus particulièrement les alkylpolyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer à l'extrait d'*Halopteris scoparia* dans la composition cosmétique, objet de la présente invention telle que définie précédemment, on peut citer les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses comme le PPG-14 laureth isophoryl dicarbamate commercialisé sous l'appellation ELFACOS™ T211, le PPG-14 palmeth-60 hexyl dicarbamate commercialisé sous l'appellation ELFACOS™ GT2125.

Comme exemples d'agents épaississants et/ou gélifiants que l'on peut associer à l'extrait d'*Halopteris scoparia* dans la composition cosmétique, objet de la présente invention telle que définie précédemment, on peut citer les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide 2-méthyl-[(1 -oxo-2- propényl)amino]-1 - propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- méthyl)acrylamido méthane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (XIII) :

$$CH_2=C(R'_3)-C(=O)-[CH_2-CH_2-O]_{n'}-R'_4 \qquad (XIII),$$

dans laquelle R'3 représente un atome d'hydrogène ou un radical méthyle, R'4 représente un radical alkyle linéaire ou ramifié comportant de huit à trente atomes de carbone et n' représente un nombre supérieur ou égal à un et inférieur ou égal à cinquante.

Les polymères de type polyélectrolytes, linéaires ou branchés ou réticulés que l'on peut associer à l'extrait d'*Halopteris scoparia* dans la composition cosmétique, objet de la présente invention telle que définie précédemment, peuvent être sélectionnés parmi les produits commercialisés sous les appellations SIMULGEL™ EG, SIMULGEL™ EPG, SEPIGEL™ 305, SIMULGEL™ 600, SIMULGEL™ NS, SIMULGEL™ INS 100, SIMULGEL™ FL, SIMULGEL™ A, SIMULGEL™ SMS 88, SEPINOV™ EMT 10, SEPIPLUS™ 400, SEPIPLUS™ 265, SEPIPLUS™ S, SEPIMAX™ Zen, SEPILIFETM NUDE, ARISTOFLEX™ AVC, ARISTOFLEX™ AVS, ARISTOFLEX™ AVL, ARISTOFLEX™ BLV, ARISTOFLEX™ Silk, ARISTOFLEX™ TAC, ARISTOFLEX™ Velvet, ARISTOFLEX™ A60, ARISTOFLEX™ ECO T, ARISTOFLEX™ PEA, ARISTOFLEX™ PEA 70, NOVEMER™ EC-1 , NOVEMER™ EC 2, ARISTOFLEX™ HMB, COSMEDIA™ SP, FLOCARE™ ET 25, FLOCARE™ ET 75, FLOCARE™ ET 26, FLOCARE™ ET 30, FLOCARE™ ET 58, FLOCARE™ PSD 30, VISCOLAM™ AT 64, VISCOLAM™ AT 100, TEXIQUETM HE 10, TEXIQUETM HE 20, TEXIQUETM PQ 37.

Comme exemples d'agents épaississants et/ou gélifiants que l'on peut associer à l'extrait d'*Halopteris scoparia* dans la composition cosmétique, objet de la présente invention telle que définie précédemment, on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ).

Comme exemples d'agents épaississants et/ou gélifiants que l'on peut associer à l'extrait d'*Halopteris scoparia* dans la composition cosmétique, objet de la présente invention telle que définie précédemment, on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme e arabique et de gomme de karaya, les glucosaminoglycanes.

Comme exemples d'agents épaississants et/ou gélifiants que l'on peut associer à l'extrait d'*Halopteris scoparia* dans la composition cosmétique, objet de la présente invention telle que définie précédemment, on peut citer la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer à l'extrait d'*Halopteris scoparia* dans la composition cosmétique, objet de la présente invention telle que définie précédemment, on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants que l'on peut associer à l'extrait d'*Halopteris scoparia* dans la composition cosmétique, objet de la présente invention telle que définie précédemment, on peut citer l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylèneglycol, le diéthylèneglycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales que l'on peut associer à l'extrait d'*Halopteris scoparia* dans la composition cosmétique, objet de la présente invention telle que définie précédemment, on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer à l'extrait d'*Halopteris scoparia* dans la composition cosmétique, objet de la présente invention telle que définie précédemment, on peut citer les xylènes sulfonates, les cumènes sulfonates, l'hexylpolyglucoside, le 2- éthylhexylpolyglucoside, le n-heptylpolyglucoside.

Comme exemples d'agents tensioactifs émulsionnants que l'on peut associer à l'extrait d'*Halopteris scoparia* dans la composition cosmétique, objet de la présente invention telle que définie précédemment, on peut citer les tensioactifs non ioniques, des tensioactifs anioniques, des tensioactifs cationiques.

Comme exemples de tensioactifs non-ioniques émulsionnants que l'on peut associer à l'extrait d'*Halopteris scoparia* dans la composition cosmétique, objet de la présente invention telle que définie précédemment, on peut citer les esters d'acides gras et de sorbitol, comme les produits commercialisés sous les appellations MONTANE™40, MONTANE™60, MONTANE™70, MONTANE™80 et MONTANE™85 ; les compositions comprenant du stéarate de glycérol et l'acide stéarique éthoxylé entre 5 moles et 150 moles d'oxyde d'éthylène, comme la composition comprenant de l'acide stéarique éthoxylé à 135 moles d'oxyde d'éthylène et du stéarate de glycérol commercialisée sous l'appellation SIMULSOL™ 165 ; les esters de mannitan ; les esters de mannitan éthoxylés ; les esters de sucrose ; les esters de méthylglucoside ; les alkylpolyglycosides comportant un radical aliphatique, linéaire ou ramifié, saturé ou insaturé, et comportant de 14 à 36 atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le 2-octyldodécyl polyxyloside, le 12-hydroxystéaryl polyglucoside ; les compositions d'alcools gras linéaires ou ramifiés, saturés ou insaturés, et comportant de 14 à 36 atomes de carbone, et d'alkylpolyglycosides tels que décrits précédemment, par exemple les compositions commercialisées sous les noms MONTANOV™68, MONTANOV™14, MONTANOV™82, MONTANOV™202, MONTANOV™S, MONTANOV™WO18, MONTANOV™L, FLUIDANOV™20X et EASYNOV™.

Comme exemples de tensioactifs anioniques émulsionnant que l'on peut associer à l'extrait d'*Halopteris scoparia* dans la composition cosmétique, objet de la présente invention telle que définie précédemment, on peut citer le glycéryl stéarate citrate, le cétéarylsulfate, les savons comme le stéarate de sodium ou le stéarate de triéthanolammonium, les dérivés N-acylés d'acides aminés salifiés, par exemple le stéaroyl glutamate.

Comme exemples de tensioactifs cationiques émulsionnants que l'on peut associer à l'extrait d'*Halopteris*

*scoparia* dans la composition cosmétique, objet de la présente invention telle que définie précédemment, on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants que l'on peut associer à l'extrait d'*Halopteris scoparia* dans la composition cosmétique, objet de la présente invention telle que définie précédemment, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture que l'on peut associer à l'extrait d'*Halopteris scoparia* dans la composition cosmétique, objet de la présente invention telle que définie précédemment, on peut citer des dérivés N-acylés d'acides aminés, comme la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV™ 14, les fibres de cellulose, les fibres de coton, les fibres de chitosane, le talc, la séricite et le mica.

Comme exemples d'agents déodorants que l'on peut associer à l'extrait d'*Halopteris scoparia* dans la composition cosmétique, objet de la présente invention telle que définie précédemment, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium ; les cyclodextrines ; les zéolithes métalliques ; le TRICLOSAN™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'huiles que l'on peut associer à l'extrait d'*Halopteris scoparia* dans la composition cosmétique, objet de la présente invention telle que définie précédemment, on peut citer les composés suivants :

- Les alcanes linéaires comportant de onze à dix-neuf atomes de carbone, comme le n-undécane, le n-dodécane, le n-tridécane, le n-tétradécane, le n-pentadécane, le n-hexadécane, le n-heptadécane, le n-octadécane, le n-nonadécane ;
- Les alcanes ramifiés, comportant de sept à quarante atomes de carbone, comme l'isododécane, l'isopentadécane, l'isohexadécane, l'isoheptadécane, l'isooctadécane, l'isononadécane ou l'isoeicosane, ou des mélanges de certains d'entre eux comme ceux cités ci-après et identifiés par leur nom INCI : C7-8 isoparaffin, C8-9 isoparaffin, C9-11 isoparaffin, C9-12 isoparaffin, C9-13 isoparaffin, C9-14 isoparaffin, C9-16 isoparaffin, C10-11 isoparaffin, C10-12 isoparaffin, C10-13 isoparaffin, C11-12 isoparaffin, C11-13 isoparaffin, C11-14 isoparaffin, C12-14 isoparaffin, C12-20 isoparaffin, C13-14 isoparaffin, C13-16 isoparaffin ;
- Les cyclo-alcanes optionnellement substitués par un ou plusieurs radicaux alkyles linéaires ou ramifiés ;
- Les huiles blanches minérales, comme celles commercialisées sous les noms suivants : Marcol™52, Marcol™82, Drakeol™6VR, Eolane™130, Eolane™150 ;
- L'hémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
- Les mélanges d'alcanes comportant de 15 à 19 atomes de carbone, lesdits alcanes étant des alcanes linéaires, des alcanes ramifiés et des cyclo-alcanes, et plus particulièrement le mélange (M1) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms Emogreen™L15 ou Emogreen™L19 ;
- Les mélanges d'alcanes linéaires comme le mélange de n-undécane et de n-tridécane comme celui commercialisé sous le nom de marque Cetiol Ultimate™ ;
- Les mélanges d'alcanes linéaires comme le mélange de n-dodécane et de n-tétradécane celui commercialisé sous le nom de marque Vegelight™ 12-14 ;
- Les éthers d'alcool gras de formule (II) :

Z1-O-Z2 (II), dans laquelle Z1 et Z2 identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther ;

Les mono-esters d'acides gras et d'alcools de formule (III) :

$$R'1-(C=O)-O-R'2 \qquad (III),$$

dans laquelle R'1-(C=O) représente un radical acyle, saturé ou insaturé, linéaire ou ramifié, comportant de huit à vingt-quatre atomes de carbone, et R'2 représente, indépendamment de R'1, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle, cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'hexyle, le palmitate d'octyle , l'oléate de méthyle, l'oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2- butyle, l'isostéarate d'hexyle, l'isostéarate d'isostéaryle ;

- Les di-esters d'acides gras et de glycérol de formule (IV) et de formule (V) :

$$R'3-(C=O)-O-CH2-CH(OH)-CH2-O-(C=O)-R'4 \qquad (IV),$$

$$R'5-(C=O)-O-CH2-CH[O-(C=O)-R'6]-CH2-OH \qquad (V),$$

dans lesquelles R'3-(C=O), R'4-(C=O), R'5-(C=O), R'6-(C=O), identiques ou différents, représentent un groupement acyle, saturé ou insaturé, linéaire ou ramifié, comportant de huit à vingt-quatre atomes de carbone ;

- Les tri-esters d'acides gras et de glycérol de formule (VI) :

$$R'7-(C=O)-O-CH2-CH[O-(C=O)-R'8]-CH2-O-(C=O)-R'9 \qquad (VI),$$

dans laquelle R'7-(C=O), R'8-(C=O) et R'9-(C=O), identiques ou différents, représentent un groupement acyle, saturé ou insaturé, linéaire ou ramifié, comportant de huit à vingt-quatre atomes de carbone ;

- Les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- Les huiles végétales éthoxylées.

Par « huiles », on entend dans la présente demande les composés et/ou les mélanges de composés insolubles dans l'eau, se présentant sous un aspect liquide à une température de 25°C.

Comme exemples de cires que l'on peut associer à l'extrait d'*Halopteris scoparia* dans la composition cosmétique, objet de la présente invention telle que définie précédemment, on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs que l'on peut associer à l'extrait d'*Halopteris scoparia* dans la composition cosmétique, objet de la présente invention telle que définie précédemment, on peut citer les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le ω-undecelynoyl phénylalanine commercialisé sous l'appellation SEPIWHITE™MSH, le SEPICALM™VG, le mono ester et/ou le diester de glycérol du ω-undecelynoyl phénylalanine, les ω-undecelynoyl dipeptides, l'arbutine, l'acide kojique, l'hydroquinone ; les composés montrant une action apaisante notamment le SEPICALM™ S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylpoglucoside ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™, la fucoxanthine ; les protéines N-acylées ; les peptides N-acylés comme le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE™ C8G, le LIPACIDE™ UG, le SEPICONTROL™ A5 ; l'OCTOPIROX™ ou le SENSIVA™ SC50 ; les composés montrant une propriété énergisante ou stimulante comme le PHYSIOGENYL™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de centalla asiatica, de fucus, de romarin, de saule ; les agents de bronzage ou de brunissement de la peau, par exemple la dihydroxyacétone (DHA), l'érythrulose, l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, par exemple les caraténoïdes ( et plus particulièrement le beta carotène et le gamma carotène), le produit commercialisé sous le nom de marque « Carrot oil » (Nom INCI : Daucus Carota, helianthus annuus Sunflower oil) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultra-violets, par exemple le produit commercialisé sous le nom de marque « SunTan Accelerator™ » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze™ (nom INCI : Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI : butylene glycol and Acetyl Tyrosine and hydrolyzed vegetable protein and Adenosine triphosphate) par la société UNIPEX, le produit commercialisé sous le nom de marque « Try-Excell™ » (nom INCI : Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze™ » (nom INCI : hydrolyzed wheat protein and acetyl tyrosine and copper gluconate) par la société Alban Muller, le produit commercialisé sous le nom de marque Tyrostan™ (nom INCI : potassium caproyl tyrosine) par la société Synerga, le produit commercialisé sous le nom de marque Tyrosinol (nom INCI : Sorbitan Isostearate, glyceryl oleate, caproyl Tyrosine) par la société Synerga, le produit commercialisé sous le nom de marque InstaBronze™ (nom INCI : Dihydroxyacetone and acetyl tyrosine and copper gluconate) commercialisé par la société Alban Muller, le produit commercialisé sous le nom de marque Tyrosilane (nom INCI : méthylsilanol and acétyl tyrosine) par la société Exymol ; les peptides connus pour leur effet d'activation de la mélanogénèse par exemple le produit commercialisé sous le nom de marque Bronzing SF Peptide powder (nom INCI : Dextran and Octapeptide-5) par la société Infinitec Activos, le produit commercialisé sous le nom de marque Melitane (nom INCI : Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions™ (nom INCI : Butylene glycol , Palmitoyl Tripeptide-40) par la société LIPOTEC, les sucres et les dérivés de sucres par exemple le produit commercialisé sous le nom de marque Tanositol™ (nom INCI : inositol) par la société Provital, le produit commercialisé sous le nom de marque Thalitan™ (ou Phycosaccharide™ AG) par la société CODIF international (nom INCI : Aqua and Hydrolyzed algin (Laminaria Digitata) and magnesium sulfate and manganese sulfate) contenant un oligosaccharide d'origine marine (acide gul023 luronique et

acide mannuronique chélatés avec les ions magnésium et manganèse), le produit commercialisé sous le nom de marque Melactiva™ (nom INCI : Maltodextrin, Mucuna Pruriens Seed extract) par la société Alban Muller, les composés riches en flavonoïdes par exemple le produit commercialisé sous le nom de marque « Biotanning » (nom INCI : Hydrolyzed citrus Aurantium dulcis fruit extract) par la société Silab et connu pour être riche en flavonoïdes de citron (de type hespéridines) ; les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis parmi ceux décrits dans la demande de brevet européen publiée sous le numéro EP 1 515 688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, des associations de mono- et diester d'acide cinnamique et de vitamine C, et plus généralement ceux cités dans la demande de brevet européen publiée sous le numéro EP 1 515 688 A2.

Comme exemples de pro-biotiques que l'on peut associer à l'extrait d'*Halopteris scoparia* dans la composition cosmétique, objet de la présente invention telle que définie précédemment, on peut citer différentes souches de Saccharomyces cerevisiae, de Bacillus cereus var toyoi, de Bacillus subtilis seul ou en combinaison avec le Bacillus licheniformis, ou encore des souches d'Enteroccocus faecium. Ces souches de microorganismes sont généralement associées à un support solide, par exemple le carbonate de calcium, le dextrose ou le sorbitol.

Comme exemples d'agents antioxydants que l'on peut associer à l'extrait d'*Halopteris scoparia* dans la composition cosmétique, objet de la présente invention telle que définie précédemment, on peut citer l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINETM GL 47S commercialisé par la société Akzo Nobel sous le nom INCI : : Tetrasodium Glutamate Diacetate.

Comme exemples de filtres solaires que l'on peut associer à l'extrait d'*Halopteris scoparia* dans la composition cosmétique, objet de la présente invention telle que définie précédemment, on peut citer tous ceux figurant dans la directive cosmétique 76/768/CEE modifiée annexe VII.

Parmi les filtres organiques solaires que l'on peut associer à l'extrait d'*Halopteris scoparia* dans la composition cosmétique, objet de la présente invention telle que définie précédemment, on peut citer la famille des dérivés de l'acide benzoïque comme les acides para-aminobenzoïques (PABA), notamment les esters de monoglycérol de PABA, les esters éthyliques de N,N25 propoxy PABA, les esters éthyliques de N,N-diéthoxy PABA, les esters éthyliques de N,Ndiméthyl PABA, les esters méthyliques de N,N-diméthyl PABA, les esters butyliques de N,Ndiméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl- 2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de pméthoxy 2-éthylhexyle), le cinnamate de p-méthoxy 2-éthoxyéthyle, le p-cinnamate de méthoxycyclohexyle, le cinnamate d'éthyl-$\alpha$-cyano-$\beta$-phényle, le cinnamate de 2-éthylhexyl-$\alpha$-cyano-$\beta$-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-5 carboxylate, la 2-hydroxy-4-n-octyloxybenzophénone, la 4-hydroxy-3-carboxybenzophénone ; le 3-(4'-méthylbenzylidène)-d,l-camphre, le 3 (benzylidène)-d,lcamphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy)-1,3,5-triazine, le 4,4-((6-(((1,1-diméthyléthyl) amino)carbonyl)phenyl)amino)-1,3,5-triazine-2,4-diyl diimino) bis-(2-éthylhexyl) ester de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-tbutylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane.

Parmi les filtres inorganiques solaires, également appelés "écrans minéraux", que l'on peut associer à l'extrait d'*Halopteris scoparia* dans la composition cosmétique, objet de la présente invention telle que définie précédemment, on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

La présente invention a également pour objet un procédé de soin cosmétique comprenant une étape d'application par voie topique sur la peau et/ou sur le cuir chevelu d'une quantité cosmétiquement efficace de l'extrait d'*Halopteris scoparia* tel que défini ci-dessus ou de la composition cosmétique le contenant telle que définie ci-dessus, pour prévenir et/ou pour réduire et/ou pour ralentir et/ou pour empêcher et/ou pour atténuer l'apparition des manifestations inesthétiques et/ou inconfortables et/ou désagréables de la peau et/ou du cuir chevelu chez l'être humain dues à la sécrétion de sébum, par exemple choisies parmi la présence des comédons fermés (points blancs ou microkystes) et/ou des comédons ouverts (points noirs) et/ou des papules et/ou des pustules et/ou des nodules et/ou des cicatrices et/ou des pores visiblement dilatés et/ou l'aspect luisant ou gras ou brillant et/ou la sensation collante.

Selon un aspect particulier de l'invention, le procédé de soin cosmétique comprend en outre une étape supplémentaire, par exemple une étape de lavage de la zone de la peau et/ou du cuir chevelu sur laquelle l'extrait d'*Halopteris scoparia* tel que défini ci-dessus ou de la composition cosmétique le contenant telle que définie ci-dessus est appliqué(e) par voie topique.

La présente invention a également pour objet un extrait d'*Halopteris scoparia* pour son utilisation par voie topique dans une méthode thérapeutique visant à diminuer la quantité de sébum, en particulier une quantité en excès de sébum, produite par la peau et/ou par le cuir chevelu chez l'être humain, en particulier dans une méthode thérapeutique permettant de prévenir et/ou de traiter les pathologies cutanées associées à l'hyperproduction de sébum, comme l'acné, les hyperséborrhées pathologiques et/ou l'eczéma séborrhéique.

Selon un aspect particulier de la présente invention, les pathologies cutanées associées à l'hyperproduction de sébum sont choisies dans le groupe constitué par l'acné, les hyperséborrhées pathologiques et/ou l'eczéma séborrhéique.

Selon un aspect particulier de la présente invention, l'extrait d'*Halopteris scoparia* utilisé dans une méthode thérapeutique visant à diminuer la quantité de sébum produite par la peau et/ou par le cuir chevelu est tel que défini ci-dessus.

Selon un aspect particulier de la présente invention, l'extrait d'*Halopteris scoparia* est présent dans une composition pharmaceutique, destinée à une application par voie topique, comprenant en outre au moins un ingrédient pharmaceutiquement acceptable. En particulier, l'extrait d'*Halopteris scoparia* est présent en tant que principe actif dans ladite composition pharmaceutique à usage topique en quantité efficace, en particulier en une teneur comprise de 0,1% massique à 30% massique, en particulier de 1% massique à 10% massique, par rapport au poids total de la composition pharmaceutique.

La présente invention a également pour objet une composition pharmaceutique à usage topique comprenant en tant que principe actif une quantité efficace d'un extrait d'*Halopteris scoparia* et au moins un ingrédient pharmaceutiquement acceptable, pour son utilisation dans une méthode thérapeutique visant à diminuer la quantité de sébum, en particulier une quantité en excès de sébum, produite par la peau et/ou par le cuir chevelu chez l'être humain, en particulier dans une méthode thérapeutique permettant de prévenir et/ou de traiter les pathologies cutanées associées à l'hyperproduction de sébum, en particulier choisies dans le groupe constitué par l'acné, les hyperséborrhées pathologiques et/ou l'eczéma séborrhéique.

La quantité efficace d'un extrait d'*Halopteris scoparia* comprise dans la composition pharmaceutique permet de diminuer la quantité de sébum, en particulier une quantité en excès de sébum, produite par la peau et/ou par le cuir chevelu chez l'être humain, en particulier l'activité anti-lipase de la peau traitée soit supérieure à 50% par rapport au témoin, et/ou que la production de lipide par les sébocytes de la peau traitée soit inhibée, et/ou que la formation du biofilm de bactéries pathogènes soit diminuée ou ralentie ou inhibée.

Selon un aspect plus particulier de la présente invention, ladite composition pharmaceutique comprend pour 100% massique :

- de 0,1% massique à 70% massique de l'extrait d'*Halopteris scoparia* tel que défini ci-dessus et
- de 30% massique à 99,9% massique d'au moins un additif technologique pharmaceutiquement acceptable.

Selon un aspect plus particulier de la présente invention, ladite composition pharmaceutique comprend pour 100% massique :

- de 0,3% massique à 60% massique de l'extrait d'*Halopteris scoparia* tel que défini ci-dessus et
- de 40% massique à 99,7% massique d'au moins un additif technologique pharmaceutiquement acceptable.

Selon un aspect plus particulier de la présente invention, ladite composition pharmaceutique comprend pour 100% massique :

- de 0,5% massique à 50% massique de l'extrait d'*Halopteris scoparia* tel que défini ci-dessus et
- de 50% massique à 99,5% massique d'au moins un additif technologique pharmaceutiquement acceptable.

Selon un aspect plus particulier de la présente invention, ladite composition pharmaceutique comprend pour 100% massique :

- de 1,0% massique à 40% massique de l'extrait d'*Halopteris scoparia* tel que défini ci-dessus et

- de 60% massique à 99,0% massique d'au moins un additif technologique pharmaceutiquement acceptable.

Selon un aspect plus particulier de la présente invention, ladite composition pharmaceutique comprend pour 100% massique :

- de 2,0% massique à 30% massique de l'extrait d'*Halopteris scoparia* tel que défini ci-dessus et

- de 70% massique à 98,0% massique d'au moins un additif technologique pharmaceutiquement acceptable.

Selon un aspect plus particulier de la présente invention, ladite composition pharmaceutique comprend pour 100% massique :

- de 3,0% massique à 20% massique de l'extrait d'*Halopteris scoparia* tel que défini ci-dessus et

- de 80% massique à 97,0% massique d'au moins un additif technologique pharmaceutiquement acceptable.

Selon un aspect plus particulier de la présente invention, ladite composition pharmaceutique comprend pour 100% massique :

- de 5,0% massique à 10% massique de l'extrait d'*Halopteris scoparia* tel que défini ci-dessus et

- de 90% massique à 95,0% massique d'au moins un additif technologique pharmaceutiquement acceptable.

Dans le cadre de l'invention, l'ingrédient pharmaceutiquement acceptable est un additif technologique habituellement mis en œuvre dans le domaine des formulations à usage topique.

Par « additif technologique », on désigne toute substance chimique ou toute composition chimique dont la fonction technique est de permettre et/ou de faciliter le mélange des différents constituants de ladite composition pharmaceutique, de faciliter et/ou d'optimiser les propriétés physiques de ladite composition pharmaceutique, comme de faciliter et/ou d'optimiser son écoulement, sa stabilité, et son incorporation dans une formulation pharmaceutique, et qui sont susceptibles de respecter les conditions requises par les réglementations en vigueur pour la mise sur le marché d'une formulation pharmaceutique.

La composition pharmaceutique à usage topique telle que définie précédemment, peut comprendre en outre, un ou plusieurs additifs technologiques choisis parmi les solvants et co-solvants, les agents d'amélioration de la pénétration cutanée.

Comme exemples de solvants et de co-solvants éventuellement présents dans les compositions pharmaceutiques à usage topique objets de la présente invention, on peut citer l'eau, les solvants organiques par exemple le glycérol, le diglycérol, les oligomères du glycérol, l'éthylène glycol, le propylène glycol, le butylène glycol, Hexylene glycol, le diéthylène glycol, le xylitol, l'erythritol, le sorbitol, les alcools hydrosolubles tels que l'éthanol, l'isopropanol ou le butanol, les mélanges d'eau et desdits solvants organiques, le propylène carbonate, l'acétate d'éthyle, l'alcool benzylique.

Comme exemples d'agents d'amélioration de la pénétration cutanée éventuellement présents dans les compositions pharmaceutiques à usage topique objets de la présente invention, on peut citer les glycols éthers comme par exemple l'éthylène glycol monométhyl éther, l'éthylène glycol mono éthyl ether, l'éthylène glycol mono propyl éther, l'éthylène glycol mono isopropyl ether, l'éthylène glycol mono butyl éther, l'éthylène glycol mono phényl éther, l'éthylène glycol mono benzyl éther, le diéthylène glycol mono méthyl éther, le diéthylène glycol mono éthyl éther et le diéthylène glycol mono-n-butyl éther, le diéthylène glycol mono éther (ou Transcutol-p), les acides gras comme l'acide oléique, les esters de glycérol d'acide gras comme par exemple le béhénate de glycérol, le palmitostéarate de glycérol, le behenoyl macroglycérides, le polyoxyethylene-2-stearyl ether, le polyoxyethylene-2-oleyl ethers, des terpenes comme par exemple le D-Limonène, des huiles essentielles comme par exemple l'huile essentielle d'eucalyptus.

De manière générale, la composition pharmaceutique selon l'invention peut comprendre des excipients et/ou des principes actifs habituellement mis en œuvre dans le domaine des formulations à usage topique, en particulier pharmaceutique ou dermopharmaceutique.

Comme exemples d'agents actifs que l'on peut associer à la composition pharmaceutique selon l'invention, on peut citer les substances ou les compositions qui apportent un effet bénéfique au sujet humain.

Ces agents actifs peuvent être par exemple des anticorps, des analgesiques, des anti-inflammatoires, des cytokines, des cytoxines, des facteurs de croissances, des hormones, des lipides, des oligonucleotides, des polymères, des polysaccharides, des polypeptides, des inhibiteurs de protease, des vitamines, des repellents d'insecte, des antibiotiques, des agents anti-fongiques.

Comme exemples d'agents analgésiques et anti-inflammatoires que l'on peut associer à la composition pharmaceutique selon l'invention, on peut citer l'acetaminophene, l'aspirine, l'acide salicylique, le methyl salicylate, la choline salicylate, le glycol salicylate, le 1-menthol, le camphre, l'acide mefenamique, l'acide fluphenamique, l'indomethacine, l'acide protizidique, le fentiazac, le tolmetin, l'acide tiaprofenique, le phenylbutazone, l'oxyphenbutazone, le clofezone, le pentazocine, le mepirizole, l'hydrocortisone, la cortisone, la dexamethasone, la fluocinolone, la triamcinolone, la medrysone, la prednisolone, la flurandrenolide, le prednisone, l'halcinonide, le methylprednisolone, le fludrocortisone, la corticosterone, la paramethasone, la betamethasone.

Comme exemples d'agents antiseptiques que l'on peut associer à la composition pharmaceutique selon l'invention, on peut citer la cetrimide, la povidone-iodine, la chlorhexidine, l'iodine, le benzalkonium chloride, l'acide benzoïque, le nitrofurazone, le peroxyde de benzoyle, l'hydrogène péroxyde, l'hexachlorophène, le phénol, le résorcinol et le cetylpyridinium chloride.

Comme exemples d'agents anti insecticides que l'on peut associer à la composition pharmaceutique selon l'invention, on peut citer le trichiorfone, le triflumerone, le fenthion, le bendiocarbe, la cyromazine, le dislubenzurone, le dicyclanile, le fluazurone, l'amitraze, la deltamethrine, la cypermethrine, le chlorfenbinphose, la flumethrine, la ivermectine, l'abermectine, l'avermectine, la doramectine, la moxidectine, la zeti-cypermethrine, la diazinone, la spinosade, l'imidaclopride, le nitenpyrane, le pyriproxysene, le sipronil, le cythioate, la lufenurone, la selamectine, la milbemycine oxime, le chlorpyrifose, le coumaphose, le propetamphose, l'alpha-cypermethrine, l'highciscypermethrine, l'ivermectine, la diflubenzurone, le cyclodiene, le carbamate and benzoyl urée.

Comme exemples d'agents anti-microbiens que l'on peut associer à la composition pharmaceutique selon l'invention, on peut citer les sulfonamides, les aminoglycosides comme par exemple le neomycine, le tobramycine, le gentamycine, l'amikacine, le kanamycine, le spectinomycine, le paromomycine, le netilmicin, les polypeptides, les cephalosporines, les oxazolidinones comme par exemple la ciprofloxacine, la levofloxacine, l'ofloxacine.

Comme exemples d'agents actifs que l'on peut associer à la composition pharmaceutique selon l'invention, on peut citer la vitamine E, le Coenzyme Q.10, la L-carnitine, la choline, l'acide folique, le magnésium et ses sels, l'acide caprylique, l'acide linoléique, l'acide laurique, la taurine, la vitamine C, la vitamine A, les vitamines du groupe B.

L'application sur la peau de l'extrait d'*Halopteris scoparia* tel que défini ci-dessus ou de la composition pharmaceutique le contenant permet :

- de limiter la formation du biofilm de bactéries opportunistes pathogènes, comme par exemple *Staphylococcus. Aureus,* sans altérer la présence de bactéries commensales telles que *Staphylococcus. Epidermidis,*
- de protéger les sébocytes de la surproduction de lipides, et
- de renforcer une activité anti-lipase de la peau ou du cuir chevelu ;

effets responsables du développement du sébum sur la peau et sur le cuir chevelu, et par conséquent des effets inesthétiques associés.

Les exemples suivant illustrent l'invention sans toutefois la limiter.

L'algue *Halopteris scoparia* sèche est broyée puis mise en contact avec un solvant composé de 90% d'eau et de 10% d'un mélange de diols composé de 5% de 1,3-propanediol (CAS 504-63-2) et 5% de 1,2-pentanediol (CAS 5343-92-0). Le ratio massique algue/solvant est de 10/90. L'ensemble est agité pendant 2 heures à 20°C. La matière végétale est ensuite séparée du solvant par centrifugation et filtration finale à 0,2 µm. Le solvant est conservé pour obtenir un extrait final liquide, ayant une matière sèche comprise entre 0,5% et 4%. Dans l'exemple de l'invention, la composition de l'extrait final obtenue est composée de 88,80% massique de l'eau, 4,90% massique de 1,3-propanediol, 4,90% massique de 1,2-pentanediol et 1,40% massique de matière sèche. L'extrait d' *Halopteris scoparia* est incorporé dans les formulations aux teneurs en masse indiquées pour les tests suivants.

A. Diminution **du** sébum et de la vitesse de regraissage *in vivo*

Principe de la méthode :

Des femmes ayant la peau mixte/grasse à grasse et un taux de sébum entre 140 et 190 µg/cm$^2$ ont été recrutées pour évaluer l'effet séborégulateur de l'extrait d'*Halopteris scoparia* versus placebo. Elles ont appliqué les produits sur leur visage deux fois par jour pendant 28 jours, avec mesure à J0 (avant application des produits) et à J28. Le taux de sébum a été mesuré avec un sébumètre.

Formules testées :

Tableau 1

| Ingredients | Placebo | Formule contenant l'extrait d'*Halopteris scoparia 1*% |
|---|---|---|
| Eau | qsp 100% | |
| Glycerin | 1% | |
| Montanov L (C14-22 Alcohols & C12-20 Alkyl Glucoside) | 1% | |
| Simulsol 165 (PEG-100 Stearate & Glyceryl Stearate) | 1% | |
| Triglyceride 5545 (Caprylic/ Caprylic Triglycerides) | 3% | |
| Montanov L (C14-22 Alcohols & C12-20 Alkyl Glucoside) | 7% | |
| Sepinov EMT10 (Hydroxyethyl Acrylate & Sodium Acryloyldimethyl Taurate Copolymer) | 0,5% | |
| Sepimax ZEN (Polyacrylate Crosspolymer-6) | 1% | |
| Extrait d'*Halopteris scoparia* (Water & Pentylene Glycol & Propanediol & Halopteris scoparia extract) | - | 1% |
| Euxyl PE9010 (Phenoxyethanol & ehtylhexylglycerin) | 1% | |
| Sensiva PA40 (Phenylpropanol & Caprylyl Glycol & Propandediol & Tocopherol) | 0,5% | |
| Neutrophil fragrance | 0,04% | |

Le taux de sébum sur la peau a été évalué avec un Sebumeter® SM 815 (Courage & Khazaka).

Il utilise une cassette munie d'un film transparent sensible aux lipides absorbés (sébum) et un capteur photométrique. Le film est appliqué sur la zone de mesure pendant 30 secondes. Sa transparence augmente proportionnellement à la quantité de sébum du film hydrolipidique avec lequel il est en contact, ce qui permet de calculer un niveau de sébum par unité de surface (en $\mu$g/cm$^2$).

Pour évaluer le taux de regraissage de la peau, la zone antérieure du front a été délipidée avec un mélange éthanol/eau (70/30) jusqu'à obtention d'une valeur de sébum $\leq$ 50 $\mu$g/cm$^2$ mesurée par Sebumeter® SM 815. Puis le taux de sébum a été à nouveau mesuré 30 minutes après la délipidation et le taux de regraissage a été calculé avec la formule suivante : (taux de sébum 30 minutes après délipidation - taux de sébum avant délipidation) / 30 minutes.

Eléments statistiques :

Les valeurs obtenues sont exprimées en moyennes +/- SEM (standard error of the mean : erreur-type de la moyenne).

Un pourcentage d'effet D28 versus D0 a été calculé :

Math 1

$$\% \text{ Effect} = \frac{\text{Moyenne (J28)} - \text{Moyenne (J0)}}{\text{Moyenne (J0)}} \text{x100}$$

L'analyse intra-groupe a été réalisée avec un test t de Student apparié si l'hypothèse de normalité était validée, sinon avec un test sur rangs de Wilcoxon.

L'analyse inter-groupe a été réalisée sur les taux de sébum (J28-J0) et sur les taux de regraissage à J28 avec un test t de Student non apparié si l'hypothèse de normalité était validée, sinon avec un test sur rangs de Wilcoxon.

Les variations étaient considérées comme statistiquement significatives lorsque la valeur p était < 0,05.

Résultats :

Le tableau 1 suivant présente les résultats sur le taux de sébum à J0 et J28 ($\mu$g/cm$^2$). Les valeurs sont exprimées en moyenne $\pm$ SEM.

Tableau 1

|  | J0 | J28 | J28-J0 | Statistiques |
|---|---|---|---|---|
| Placebo | 170,8 ± 2,8 | 136,9 ± 6,8 | -33,9 ± 7,9 (-20%) | p < 0,001 vs J0 |
| *Halopteris scoparia 1%* | 169,4 ± 3,7 | 111,2 ± 9,5 | -58,2 ± 9,4 (-34%) | p < 0,001 vs J0<br>p = 0,044 vs placebo |

Le tableau 2 suivant présente les résultats sur le taux de regraissage à J0 et J28 ($\mu$g/cm$^2$/h). Les valeurs sont exprimées en moyenne ± SEM.

Tableau 2

|  | J0 | J28 | J28-J0 | Statistiques |
|---|---|---|---|---|
| Placebo | 87,9 ± 7,8 | 71,0 ± 7,9 | -17,0 ± 9,5 (-19%) | p=0,09 vs J0 |
| *Halopteris scoparia 1%* | 72,8 ± 7,0 | 40,7 ± 4,2 | -32,1 ± 8,8 (-44%) | p=0,002 vs J0<br>p=0,003 vs placebo |

**B. Production de sébum par des sébocytes en conditions inflammatoires**

Principe de la méthode :

Des sébocytes caucasiens issus de la technologie hiPSc (human induced pluripotent stem cells) sont directement décongelés en plaque 96 puits, préalablement revêtues avec de la fibronectine, et cultivés pendant 3 jours dans un milieu de culture adapté et supplémenté d'un complément permettant leur maturation. Ils sont ensuite co-traités avec les ingrédients à tester +/- acide arachidonique à 15$\mu$M pendant 48h. Chaque condition est réalisée en quadriplicat (n=4).
Les sébocytes sont fixés, perméabilisés et marqués avec la sonde BodiPy (lipides) et le Hoechst 33342 (noyaux).
Les cellules sont ensuite imagées à l'aide d'un microscope automatisé (CellInsight CX5) en 20X et 24 champs sont acquis par puits. L'intensité de fluorescence est ensuite quantifiée et normalisée à la quantité de noyaux.

Eléments statistiques :

Les valeurs obtenues ont été exprimées en moyennes +/- SD (standard deviation : écart type). Pour chaque condition, un pourcentage d'effet a été calculé :

Math 2

$$\% \text{ Effect} = \frac{\text{Moyenne (X)} - Moyenne\ (cellules\ stressées)}{Moyenne\ (cellules\ stressées)} \text{x}100$$

X: condition étudiée
Cellules stressées: sébocytes traités avec l'acide arachidonique

Un pourcentage de protection a également été calculé :

Math 3

$$\% \text{ Protection}$$
$$= \frac{\text{Moyenne (X)} - Moyenne\ (cellules\ stressées)}{Moyenne\ (cellules\ non\ stressées) - Moyenne\ (cellules\ stressées)} \text{x}100$$

X: condition étudiée
Cellules non stressées: sébocytes non traités (condition témoin)
Cellules stressées: sébocytes traités avec l'acide arachidonique

L'analyse statistique a été réalisée par un test de Student et un seuil de significativité fixé à 5%.

Résultats :

Le tableau 3 suivant présente les résultats de l'effet de l'extrait d'*Halopteris scoparia* sur la surproduction de lipides induite par l'acide arachidonique sur sébocytes caucasiens issus d'iPS.

Tableau 3

|  | Moyenne ± SD (Unité Arbitraire) | % effet | % protection |
|---|---|---|---|
| Cellules non traitées (contrôle) | 70 ± 40 | - | - |
| + acide arachidonique (15µM) | 10944 ± 1984 | +15610%** vs cellules non traitées | - |
| + *Halopteris scoparia* 0,1% | 5041 ± 4170 | -54%* vs cellules stressées | 54%** |
| **: p<0,01 | | | |

L'analyse des clichés obtenus après le marquage Bodipy montre qu'après 48h d'incubation, l'acide arachidonique stimule la production de lipides par les sébocytes (+15610%, p<0,01).
L'extrait d'*Halopteris scoparia* diminue la surproduction de lipides induite par l'acide arachidonique de 54% (p<0,01).

**C. Production de sébum par des sébocytes induite par les vésicules extracellulaires (VEs) de C. *acnes***

Principe de la méthode :

Les sébocytes caucasiens issus d'iPS (Phenocell) sont décongelés et ensemencés directement en plaque 48 puits préalablement coatées avec de la fibronectine à 27 500 cellules/puits.
Les cellules sont ensuite incubées 3 jours à 37°C sous 5% de $CO_2$ puis traitées pendant 24h avec l'extrait d'*Halopteris scoparia* à 0,1%.
Puis les cellules sont mises en contact avec les vésicules extracellulaires (VEs) issues de la souche virulente DSM1897 de C. *acnes* (26 µg/mL) pendant 48 heures.
Un marquage Bodipy est réalisé afin d'étudier l'influence des VEs sur la production de lipides.

Eléments statistiques :

Les valeurs obtenues sont exprimées en moyennes +/- SD (standard deviation : écart type). Pour chaque condition, un pourcentage d'effet a été calculé :

Math 2

$$\% \text{ Effect} = \frac{\text{Moyenne (X)} - Moyenne\ (cellules\ stressées)}{Moyenne\ (cellules\ stressées)} \text{x}100$$

X: condition étudiée
Cellules stressées: sébocytes traités avec les VEs

Un pourcentage de protection a également été calculé :

Math 3

$$\% \text{ Protection}$$

$$= \frac{\text{Moyenne (X)} - Moyenne\ (cellules\ stressées)}{Moyenne\ (cellules\ non\ stressées) - Moyenne\ (cellules\ stressées)} \text{x}100$$

X: condition étudiée
Cellules non stressées: sébocytes non traités (condition témoin)
Cellules stressées: sébocytes traités avec les VEs

L'analyse statistique a été réalisée par un test de Student avec un seuil de significativité fixé à 5%.

Résultats :

Le tableau 4 suivant présente les résultats sur l'effet de l'extrait d'*Halopteris scoparia* sur la surproduction de lipides induite par les VEs de C. *acnes* sur sébocytes caucasiens issus d'iPS.

Tableau 4

|  | Moyenne ± SD (Unité Arbitraire) | % effet | % protection |
|---|---|---|---|
| Cellules non traitées (contrôle) | 128 ± 36 | - | - |
| + VEs DSM1897 (26 μg/mL) | 316 ± 48 | +146%** vs cellules non traitées | - |
| + *Halopteris scoparia* 0,1% | 114 ± 43 | -64%** vs cellules stressées | 108%** |
| **: p<0,01 | | | |

L'analyse des clichés obtenus après le marquage Bodipy montre qu'après 48h d'incubation, les vésicules extracellulaires de C. *acnes* stimulent la production de lipides par les sébocytes (+146%, p<0,01).
L'extrait d'*Halopteris scoparia* inhibe la surproduction de lipides induite par les VEs de C. *acnes* de 64% (p<0,01).

**D. Respect du microbiote cutané**

Principe de la méthode :

La méthodologie du test est décrite ci-dessous :
Une co-culture (consortium) des cinq principales bactéries du microbiote du visage *(Cutibacterium acnes, Staphylococcus epidermidis, Streptococcus mitis, Micrococcus luteus* et *Corynebacterium xerosis)* est réalisée à partir d'isolats bactériens sauvages. Les bactéries sont dans des proportions représentatives du microbiote de la joue. Le consortium est incubé dans des conditions imitant celles rencontrées sur la peau (pH faible, température basse, faible concentration en nutriments), pendant 8 heures (temps de contact maximal d'un produit cosmétique).
Après huit heures de contact avec un extrait d'*Halopteris scoparia,* les concentrations bactériennes sont les mêmes que dans le contrôle (consortium sans aucun produit), indiquant que l'un extrait d'*Halopteris scoparia* n'a pas d'impact significatif négatif sur les principales bactéries cutanées et peut être considéré comme « respectueux du microbiote ».
Les souches bactériennes suivantes (souches commensale du microbiote cutané) ont été sélectionnées et utilisées pour réaliser un consortium calibré :

- *Cutibacterium acnes,* 58 %
- *Staphylococcus epidermidis,* 18 %
- *Streptococcus sp., 14,6 %*
- *Micrococcus luteus, 4,4 %*
- *Corynebacterium xerosis, 5 %*

Ce consortium a été mis en contact pendant 8h avec l'extrait d'*Halopteris scoparia* 1% et les concentrations de chaque espèce ont été mesurées. La réduction logarithmique (RL) a été calculée par rapport au contrôle (bactéries sans extrait d'*Halopteris scoparia)* pour chaque espèce bactérienne, telle que :

Math 4

$$RL_{espèce\ X} = (\log\ UFC/mL\ pour\ le\ produit\ testé) - (\log\ UFC/mL\ contrôle)$$

L'impact de l'extrait a été mesuré grâce à l'établissement d'un score, qui est égal à l'addition des RL obtenues pour les 5 espèces bactériennes :

Math 5

$$Score = RL_{C.\ acnes} + RL_{S.\ epidermidis} + RL_{S.\ mitis} + RL_{M.\ luteus} + RL_{C.\ xerosis}$$

Une réduction logarithmique réduction inférieure ou égale à 1 (RL ≤ 1) n'est pas considérée significative. Donc, un score compris entre +5 et -5 indique un produit neutre pour le microbiote (respectueux du microbiote).

Résultats :

Les réductions logarithmiques obtenues pour chaque espèce étaient de :

- $RL_{C.\ acnes}$ = -0,54
- $RL_{S.\ epidermidis}$ =0,35
- $RL_{S.\ mitis}$ = 0,0
- $RL_{M.\ luteus}$ = -0,32
- $RL_{C.\ xerosis}$ = -0,12

Le score obtenu pour l'extrait d'*Halopteris scoparia* selon ce modèle est donc -0,63, qui signifie qu'il n'y a pas de modification du consortium microbien en présence de l'extrait d'*H. scoparia.* Ce score étant compris entre -5 et +5, l'extrait est considéré comme respectueux du microbiote.

Conclusion :

Tableau 5

|  | Résultats |
|---|---|
| Clinique | Diminution du sébum et de la vitesse de regraissage |
| Production lipides par sébocytes | Diminution de la production de lipides |
| Effets des VEs de C. *acnes* sur production de lipides | Diminution de la production de lipides |
| Respect du microbiote cutané | Pas d'impact sur les souches commensales de la joue |

**Revendications**

1. Utilisation par voie topique d'un extrait d'*Halopteris scoparia* comme principe actif cosmétique pour prévenir et/ou pour réduire et/ou pour ralentir et/ou pour empêcher et/ou pour atténuer la présence de comédons fermés et/ou de comédons ouverts et/ou de papules et/ou de pustules et/ou de nodules et/ou de cicatrice et/ou de pore visiblement dilaté et/ou d'un aspect luisant ou gras ou brillant et/ou d'une sensation collante de la peau et/ou du cuir chevelu chez l'être humain dues à une sécrétion de sébum d'une peau essentiellement saine.

2. Utilisation selon la revendication 1, **caractérisée en ce que** l'extrait d'*Halopteris scoparia* comprenant pour 100% de sa masse :

    - De 80% massique à 95% massique d'eau ;
    - De 4,5% massique à 15,0% massique d'un solvant organique choisi parmi les éléments du groupe constitué par le 1,2-propanediol, le 1,3-propanediol, le 1,4-butanediol, le 1,3-butanediol, le 1,2-butanediol, le 1,2-pentanediol, le 2-méthyl-2,4-pentanediol, le 1,6-hexanediol, le 1,8-octanediol, et d'un mélange de ces composés ;
    - De 0,5% massique à 5,0% massique de matière sèche.

3. Utilisation selon l'une quelconque des revendications 1 ou 2, **caractérisée en ce que** le solvant organique est un mélange de 1,3-propanediol et de 1,2-pentanediol.

4. Utilisation selon l'une quelconque des revendications 1 à 3, **caractérisée en ce que** le solvant organique comprend pour 100% de sa masse, le solvant organique est un mélange de deux diols différents l'un de l'autre en proportion massique égale.

5. Utilisation selon l'une quelconque des revendications 1 à 4, **caractérisée en ce que** l'extrait d'*Halopteris scoparia* est obtenu par un procédé comprenant les étapes suivantes :

- un étape a) de séchage et de broyage de l'algue *Halopteris scoparia* de façon à obtenir une biomasse ;
- un étape b) de mise en contact de la biomasse obtenue à l'étape a) avec ledit solvant organique de façon à obtenir un mélange, ledit mélange comprenant pour 100% de sa masse, de 5% massique à 15% massique de la biomasse et de 85% massique à 95% massique dudit solvant organique ;
- un étape c) d'agitation du mélange obtenu à l'étape b) pendant une durée comprise entre 1 heure et 6 heures, à une température comprise entre 15°C et 35°C ;
- un étape d) de séparation de la biomasse du mélange obtenu à l'issue de l'étape c)

pour isoler une phase liquide qui correspond à l'extrait d'*Halopteris scoparia.*

6. Utilisation selon l'une quelconque des revendications 1 à 5, **caractérisée en ce que** les manifestations inesthétiques et/ou inconfortables et/ou désagréables de la peau et/ou du cuir chevelu chez l'être humain dues à la sécrétion de sébum sont choisies parmi la présence des comédons fermés (points blancs ou microkystes) et/ou des comédons ouverts (points noirs) et/ou des papules et/ou des pustules et/ou des nodules et/ou des cicatrices et/ou des pores visiblement dilatés et/ou l'aspect luisant ou gras ou brillant et/ou la sensation collante.

7. Utilisation selon l'une quelconque des revendications 1 à 6, **caractérisée en ce que** l'extrait d'*Halopteris scoparia* est présent dans une composition cosmétique à usage topique comprenant en outre au moins un ingrédient cosmétiquement acceptable, l'extrait d'*Halopteris scoparia* étant présent dans la composition cosmétique en une teneur comprise de 0,01% massique à 5,00% massique, en particulier 1% massique, par rapport au poids total de la composition.

8. Extrait d'*Halopteris scoparia* sous une forme adaptée pour une application par voie topique pour son utilisation thérapeutique permettant de prévenir et/ou de traiter les pathologies cutanées associées à l'hyperproduction de sébum, comme l'acné, les hyperséborrhées pathologiques et/ou l'eczéma séborrhéique, l'extrait d'*Halopteris scoparia* étant obtenu par un procédé comprenant les étapes suivantes :

- un étape a) de séchage et de broyage de l'algue *Halopteris scoparia* de façon à obtenir une biomasse ;
- un étape b) de mise en contact de la biomasse obtenue à l'étape a) avec ledit solvant organique de façon à obtenir un mélange, ledit mélange comprenant pour 100% de sa masse, de 5% massique à 15% massique de la biomasse et de 85% massique à 95% massique dudit solvant organique ;
- un étape c) d'agitation du mélange obtenu à l'étape b) pendant une durée comprise entre 1 heure et 6 heures, à une température comprise entre 15°C et 35°C ;
- un étape d) de séparation de la biomasse du mélange obtenu à l'issue de l'étape c) pour isoler une phase liquide qui correspond à l'extrait d'*Halopteris scoparia.*

9. Extrait d'*Halopteris scoparia* selon la revendication 8, **caractérisée en ce que** l'extrait d'*Halopteris scoparia* comprend pour 100% de sa masse :

- De 80% massique à 95% massique d'eau ;
- De 4,5% massique à 15,0% massique d'un solvant organique choisi parmi les éléments du groupe constitué par le 1,2-propanediol, le 1,3-propanediol, le 1,4-butanediol, le 1,3-butanediol, le 1,2-butanediol, le 1,2-pentanediol, le 2-méthyl-2,4-pentanediol, le 1,6-hexanediol, le 1,8-octanediol, et d'un mélange de ces composés ;
- De 0,5% massique à 5,0% massique de matière sèche.

10. Extrait d'*Halopteris scoparia* selon l'une quelconque des revendications 8 ou 9, **caractérisée en ce que** l'extrait est présent dans une composition pharmaceutique en une teneur comprise de 0,1% massique à 30% massique par rapport au poids total de la composition, la composition comprenant en outre au moins un ingrédient pharmaceutiquement acceptable.

11. Composition pharmaceutique comprenant en tant que principe actif une quantité efficace d'un extrait d'*Halopteris scoparia* tel que défini dans l'une quelconque des revendications 8 à 10 et au moins un ingrédient pharmaceutiquement acceptable, ladite composition étant sous une forme adaptée pour une application par voie topique pour son utilisation thérapeutique permettant de prévenir et/ou de traiter les pathologies cutanées associées à l'hyperproduction de sébum, comme l'acné, les hyperséborrhées pathologiques et/ou l'eczéma séborrhéique.

Europäisches
Patentamt

European
Patent Office

Office européen
des brevets

## RAPPORT DE RECHERCHE EUROPEENNE

Numéro de la demande

EP 25 22 5219

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 2006/134131 A1 (GEDOUIN ANTOINE [FR] ET AL) 22 juin 2006 (2006-06-22) <br> * alinéa [0001] * <br> * alinéa [0006] * <br> * tableaux 1, 2 * <br> * revendications * <br> ----- | 1-11 | INV. <br> A61K8/9706 <br> A61Q19/00 <br> A61K8/9711 |
| A | FR 2 837 386 A1 (GELYMA [FR]) 26 septembre 2003 (2003-09-26) <br> * page 1, ligne 8 - ligne 22 * <br> * page 2, ligne 1 - ligne 3 * <br> * exemples * <br> * revendications * <br> ----- | 1-11 | |
| A | CN 118 787 573 A (GUANGZHOU DAIANDI BIOLOGICAL TECH CO LTD) 18 octobre 2024 (2024-10-18) <br> * abrégé * <br> ----- | 1-11 | |
| A | Bedoux Gilles ET AL: "Chapter 12: Bioactive Components from Seaweeds" In: "ADVANCES IN BOTANICAL RESEARCH", 1 janvier 2014 (2014-01-01), LONDON, GB, XP055869490, ISSN: 0065-2296 vol. 71, pages 345-378, DOI: 10.1016/B978-0-12-408062-1.00012-3, * tableau 12.1 * <br> ----- <br> -/-- | 1-11 | **DOMAINES TECHNIQUES RECHERCHES (IPC)** <br> A61K <br> A61Q |

Le présent rapport a été établi pour toutes les revendications

| Lieu de la recherche | Date d'achèvement de la recherche | Examinateur |
|---|---|---|
| Munich | 22 mai 2026 | Irwin, Lucy |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

    .......................................................................................
& : membre de la même famille, document correspondant

EPO FORM 1503 03.82 (P04C02)

page 1 de 2

Europäisches
Patentamt

European
Patent Office

Office européen
des brevets

**RAPPORT DE RECHERCHE EUROPEENNE**

Numéro de la demande

EP 25 22 5219

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | JANUÁRIO ADRIANA P. ET AL: "Red Seaweed-Derived Compounds as a Potential New Approach for Acne Vulgaris Care", PHARMACEUTICS, vol. 13, no. 11, 15 novembre 2021 (2021-11-15), page 1930, XP093212559, Switzerland ISSN: 1999-4923, DOI: 10.3390/pharmaceutics13111930 * Voir la "Introduction", page 1 ainsi que la partie 3 "Pathophysiological Targets for the management of Acne Vulgaris", page 3. * | 1-11 | |
| A | Nn: "Advanced Cosmetic Active Agents Based on the Power of Algae", , 1 janvier 2020 (2020-01-01), pages 1-16, XP055658581, ULprospector Extrait de l'Internet: URL:https://www.ulprospector.com/documents/1253668.pdf?bs=15394&b=564817&st=20&r=eu&ind=personalcare [extrait le 2020-01-16] * "Actiseane"; page 2 * | 1-11 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 22 mai 2026 | Irwin, Lucy |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

....................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 25 22 5219

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-05-2026

| Document brevet cité au rapport de recherche | Date de publication | Membre(s) de la famille de brevet(s) | | Date de publication |
|---|---|---|---|---|
| US 2006134131 A1 | 22-06-2006 | AT | E344654 T1 | 15-11-2006 |
| | | EP | 1536751 A2 | 08-06-2005 |
| | | FR | 2844449 A1 | 19-03-2004 |
| | | JP | 2006501266 A | 12-01-2006 |
| | | US | 2006134131 A1 | 22-06-2006 |
| | | WO | 2004024102 A2 | 25-03-2004 |
| FR 2837386 A1 | 26-09-2003 | AUCUN | | |
| CN 118787573 A | 18-10-2024 | AUCUN | | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9600719 A
- EP 0971683 A
- EP 1515688 A2

**Littérature non-brevet citée dans la description**

- Human Microbiome Project. National Institute of Health, 2007
- *CHEMICAL ABSTRACTS*, 3891-98-3
- *CHEMICAL ABSTRACTS*, 504-63-2
- *CHEMICAL ABSTRACTS*, 5343-92-0